Europäisches Patentamt

**European Patent Office**

**Office européen des brevets**

(19)

(11) Publication number: **0 167 057**
**A2**

(12) # EUROPEAN PATENT APPLICATION

(21) Application number: **85107536.6**

(22) Date of filing: **18.06.85**

(51) Int. Cl.⁴: **A 61 K 47/00**

(30) Priority: **29.06.84 US 626523**

(43) Date of publication of application:
**08.01.86 Bulletin 86/2**

(84) Designated Contracting States:
**AT BE CH DE FR GB IT LI LU NL SE**

(71) Applicant: **NELSON RESEARCH & DEVELOPMENT COMPANY**
**1001 Health Sciences Road West**
**Irvine, CA 92715(US)**

(72) Inventor: **Roberts, Eugene**
**138 Seymour Place**
**Monrovia California 91016(US)**

(74) Representative: **Baillie, Iain Cameron et al,**
**c/o Ladas & Parry Isartorplatz 5**
**D-8000 München 2(DE)**

(54) Composition for use in a method for promoting nerve regeneration.

(57) A method for promoting regeneration of damaged nerve tissue, comprising administering, either alone or in combination, an effective amount of an antimitotic agent or a proton-withdrawing buffer to the damage site. Antimitotic agents reduce the rate of growth of glial cells, and buffers facilitate the growth of nerve tissue and inhibit glial cell growth. Referred antimitotic agents are cytosine arabinoside, 5-fluorouracil, and hydroxyurea. Preferred buffers are TREA and HEPES. Compositions are disclosed which include antimitotic agent, buffer, and an oxygen-supplying compound, such as hydrogen peroxide.

EP 0 167 057 A2

# COMPOSITION FOR USE IN A METHOD FOR PROMOTING NERVE REGENERATION

## Background of the Invention

This invention relates to compositions for use in a method for promoting nerve regeneration.

Neurons are postmitotic cells which do not undergo cell division or mitosis and accordingly are resistant to antimitotic agents. Neurons are closely associated with and surrounded by glial cells or astrocytes which proliferate and are susceptible to antimitotic agents. One of the difficulties in achieving regeneration of nerve fibers after they have been damaged or severed is that the glial cells proliferate and form a barrier to the regenerating nerve fibers. The result is that the further movement of the fibers toward anticipated attachment sites is blocked and regeneration of structure and function ceases.

Oxygen is vital to the normal function and development of nerves. If oxygen can be increased, this will favor new growth. As demonstrated by R. Llinas, et al., Fed. Proc. 40, #8, 2240-5 (1981), $H_2O_2$ in mammalian Ringer's solution can favor nerve survival and vitality.

## Summary of the Invention

According to the invention there is provided a pharmaceutical composition for use in a method of promoting nerve regeneration, comprising: a pharmacologically accepted carrier and an antimitotic agent. The invention also embraces the use of an antimitotic agent in the preparation of such a composition and in the preparation of medicaments for promoting the extension of neuritic processes.

In accordance with one embodiment of the present invention, it has now been discovered that antimitotic agents may be used to modulate the growth of glial cells. By administering an effective amount of antimitotic agent to the site of nerve damage or injury, the growth of glial cells can be inhibited, permitting unimpeded growth of nerve tissue.

Accordingly, one aspect of the present invention is a method for promoting regeneration of damaged nerve tissue in a mammal (such as a human), comprising administering an effective amount of antimitotic agent to the damage site. The appropriate amount of antimitotic agent is an amount sufficient to reduce the rate of growth of glial cells to the extent that glial cell growth does not prevent nerve tissue growth. The antimitotic agent is administered in a concentration of about 3 to about 20 micromolar. Preferred antimitotic agents are cytosine arabinoside, 5-fluorouracil, hydroxyurea, and methotrexate.

Another embodiment of the invention provides a pharmaceutical composition for use in a method of promoting nerve regeneration comprising a pharmacologically acceptable carrier and a buffer providing said composition with a pH of from 7 to 8. The invention also embraces the use of such a buffer in the manufacture of a medicament for promoting extension of neuritic processes.

-3-

In areas of nerve injury where blood supply is limited and edema develops, an excess of protons and low pH usually occurs. It has now been discovered that proton-withdrawing substances, herein generically referred to as "buffers", serve to inhibit growth of glial tissue when used to remove protons and increase the pH at the site of a nerve injury. In addition, such buffers unexpectedly promote membrane fluidity, metabolic transport, transport of - aminobutyric acid (GABA), calcium transport, and other membrane functions in neural tissue. It has also now been discovered that, as an apparent result of these membrane- enhancing properties, buffers facilitate and promote the growth and repair of damaged nerve tissue and promote the availability of oxygen at the damage site.

The present invention also includes a method for promoting regeneration of damaged nerve tissue in a mammal, comprising the step of increasing the pH at the damage site to between about 7 and 8, and preferably to about 7.3. The pH is increased by administering a buffer (proton-withdrawing agent) to the damage site. The buffer is preferably administered in a solution or composition having a buffer concentration of between about 0.5 and about 20 millimolar, and preferably between about 1.5 and about 8 millimolar. Preferred buffers or proton-withdrawing substances include HEPES and TREA. This embodiment of the invention is also useful for promoting the growth of undamaged nerve tissue. Thus the buffer-containing compositions in this invention

-3a-

may be used to promote growth of undamaged nerve tissue.

In the preferred embodiment of the present invention, the method for promoting regeneration of damaged nerve tissue comprises administering both an antimitotic agent and a buffer to the damage site. It is also preferred that an oxygen-supplying substance, such as any of the pharmacologically-acceptable peroxides, and particularly hydrogen peroxide, be administered with the buffer and/or antimitotic agent to the damage site. An appropriate concentration for hydrogen peroxide is 0.002% to 0.005% by volume.

The present invention also includes pharmaceutical compositions for use in the method of promoting regeneration of damaged nerve tissue, comprising a pharmacologically-acceptable carrier and an effective amount of an antimitotic agent. The preferred concentration of the antimitotic agent in the carrier concentration is about 3 to about 20 micromolar. Preferred antimitotic agents are cytosine arabinoside, 5-fluorouracil, hydroxyurea, and methotrexate.

Another composition falling within the present invention is a pharmaceutical composition for promoting regeneration of damaged nerve tissue, comprising a pharmacologically-acceptable carrier and a buffer, preferably HEPES or TREA, preferably having a concentration between about 0.5 and about 20 millimolar.

-3b-

A preferred composition according to the present invention includes both an antimitotic agent and a buffer in the concentrations and of the types described above.

The compositions of the present invention may also include an oxygen-supplying compound, such as hydrogen peroxide, preferably in a concentration of from about 0.002% to about 0.005% by volume.

Through use of the methods and compositions of the present invention, the growth of neurons and glial cells can be modulated by buffers and antimitotic agents applied under suitable conditions and growth of nerve fibers can be directed in an orderly fashion to achieve nerve regeneration. In situations where there is great nerve damage or transection, whether in central or peripheral nervous systems, conditions are developed which inhibit the proliferation of glial cells sufficiently to block

-4-

extensive growth yet allow adequate growth for scaffolding on which orderly development can proceed, and favor the regeneration of nerve fibers.

## Detailed Description of the Invention

Suitable antimitotic agents may be selected, e. g., from the various folate inhibitors, such as methotrexate; pyrimidine analogs, such as cytosine arabinoside, 5-fluorouracil, floxuridine, N-phosphonoacetyl-L-aspartate, azauridine, azaribine, and idoxuridine; purine analogs, such as mercaptopurine and thioguanine; alkylating agents, such as nitrogen mustards, dacarbazine, carmustine, lomustine, and semustine; antibiotics, such as dactinomycin, daunorubicin, doxorubicin, and bleomycins; and other antiproliferative agents, such as cisplatin, hydroxyurea, and guanazole. Particularly preferred antimitotic agents are hydroxyurea, cytosine arabinoside, methotrexate, and 5-fluorouracil.

Buffers (proton-withdrawing compounds) useful in increasing pH and thereby inhibiting or regulating the growth of glial cells include:
ACES, 2[2-amino-2-oxoethyl)-amino] ethanesulfonic acid; ADA, N-(2-acetamido)-2-iminodiacetic acid; AEPD, 2-amino-2-ethyl-1, 3, -propanediol; AMP, 2-amino-2-methyl-1-propanol; AMPD, 2-amino-2-methyl-1, 3-propanediol; BES, N, N-bis (2-hydroxyethyl)-2-aminoethanesulfonic acid; BICINE, N,N-bis(2-hydroxyethyl)-glycine; BIS-TRIS, bis(2-hydroxyethyl)-imino-tris(hydroxymethyl) methane; BIS-TRIS PROPANE, 1,3,bis[tris(hydroxymethyl)-methylamino[propane; DEA, diethanolamine; EPPS, N-(2-hydroxyethyl)-1-piperazinepropanesulfonic acid; HEPES, N-2-hydroxyethylpiperazine-N'-2-ethanesulfonic acid; MEA, monoethanol-amine; MES, 2-(N-morpholino)ethanesulfonic acid; MOPS, 3-(N-morpholino) propanesulfonic acid; PIPES, piperazine-N,N'-bis(2-ethanesulfonic acid);

TAPS, tris(hydroxymethyl)methylaminopropanesulfonic acid; TES, N-tris(hydroxymethyl)-methyl-2-aminoethanesulfonic acid; TREA, triethanolamine; TRICINE, N-tris(hydroxymethyl)methylglycine; and TRIS, tris(hydroxymethyl)aminomethane.

Preferred buffers are TREA, HEPES, and TRIS.

The amounts of these substances considered useful as promoters of nerve regeneration varies according to the concentration necessary to inhibit glial cell growth, but would generally be in the range of 3 to 20 micromolar for antimitotic agents, and in the range of 0.5 to 20 millimolar for buffers and other proton withdrawing substances.

The efficacy of various buffers at pH 7.3 (physiological pH) on a molar basis is directly related to the concentration of the unprotonated form. This accounts for differences between buffers with different values of pKa on a total molar basis, those with higher values being less effective because of smaller concentration of unprotonated form at any given pH. The concentrations of unprotonated form found to be effective are between $1 \times 10^{-5}$ M and $1 \times 10^{-3}$ M, preferably around $2-8 \times 10^{-4}$ M.

Pharmaceutical compositions suitable for application to humans and animals with damaged or severed nerves include sterile isomolar preparations of antimitotic agents with representative proton scavengers and buffers. The antimitotic agents would normally be available in the range of 3 to 20 micromolar and would preferably be buffered to a pH of 7-8, with a pH 7.3 considered optimal.

The pharmaceutical compositions may be in the form of solutions, gels, aqueous or oily suspensions, emulsions, creams and powders. Sterile isotonic saline solution may advantageously be used as a vehicle for the compositions of the present invention.

It is important that these compositions be administered to the damage site. From the foregoing

-6-

discussion, it is apparent that the compositions of the present invention can be administered topically, transdermally, intrathecally, by injection, and by slow perfusion through silicone tubing.

Example 1:  The Effects of TREA, HEPES, TRIS, and Cytosine Arabinoside on Chick Embryo Peripheral Ganglia in Vitro

Methods

The simplest model in which to test the effects of nerve-growth promoting substances are excised chick embryo ganglia maintained in tissue culture.  These ganglia are known to respond to nanomolar concentrations of nerve growth factor (NGF).

Dorsal root ganglia (DRG), trigeminal, and sympathetic ganglia from 7-8 day chick embryos were dissected in Dulbecco's phosphate buffered saline (Gibco), and cultured in the presence of different concentrations of triethanolamine (TREA), HEPES and TRIS buffers.  It was determined that concentrations of 2.5 and 5 mM of each buffer stimulated the maximal response of neurite extension.  Thereafter, DRG were used exclusively for the rest of the experiments.

Six DRG were placed in individual 60 mm culture dishes (Falcon 3002).  In the normal culture condition series, 5 ml complete medium was added to each dish.  This medium contained 85% Dulbecco's Modified Eagles' Medium, 10% dialyzed fetal bovine serum (both from Gibco), 3% glucose (600 mg% final), 1% glutamine (200 mM final) and 1% penicillin-streptomycin mix (Gibco).  Each set of experiments which was repeated 3-4 times consisted of 2-4 dishes per treatment.  All dishes were incubated for 3 days at 39°C.  For each day's experiment, a control group (untreated) and a group treated with nerve growth factor (NGF) at a final concentration of 10  nM were run in parallel with the buffer-treated groups.

-7-

Each buffer was prepared as a 10 X stock solution by dissolving the buffer in complete medium and adjusting the pH to 7.2-7.4. In every series, TREA, HEPES, and TRIS were tested at final concentrations of 2.5 mM and 5.0 mM.

A series of ganglia also was run by adding cytosine arabinoside (ara C; Cytosar, Upjohn) at a final concentration of 27 micromolar in the complete medium. The various buffers, or NGF were added, or direct current applied, to the DRG in this ara C media.

Fiber Index: Neurite Outgrowth

Neurite outgrowth was determined in cultures after 3 days in vitro after fixation in 3.5% glutaraldehyde. The scoring technique is that of Fenton, E.L., Exp. Cell Res. 50:383 (1970). The scoring system ranges from 0 to +5 and is based on the neurite outgrowth observed in standard NGF cultures. Using NGF, most observers note the maximal response after 3-6 days in vitro; the response is more variable at 9 days. However, we have used this later time point in experiments in which we determined long term effects with a Cajal silver strain to substantiate phase microscopy scoring of the neurite outgrowth.

The first experiments involved the culturing of various peripheral ganglia in the presence of concentrations of TREA from 1-10 mM. It was determined that trigeminal, dorsal root, and sympathetic ganglia all responded by elaborating neuritic processes at concentrations of 2.5 and 5 mM. In contrast to control trigeminal ganglia, trigeminal ganglia cultures in 5 mM TREA or 2.5 mM TREA and sympathetic ganglia cultured in 2.5 mM TREA all demonstrated long, filamentous neuritic processes extending from the centrally-located neuronal cell bodies out to and beyond the mat of underlying non-neuronal cells. Semi-quantitative assessment of neurite outgrowth was determined in the series of experiments conducted on dorsal root ganglia in complete media. The scoring of these cultures was: control, +1 response; NGF,

-8-

+5 response; 5 mM TREA, +3.5 response. The data obtained fron the entire series are summarized in Table 1. In all cases, significance of effect was determined using a Dunnett's Multicomparison of Treatment Means with a control test with a p limit of 0.01. Significant increase in neurite extension was obtained with all treatments.

Constant incubation in the presence of cytosine arabinoside (ara C) significantly increased the neurite outgrowth in control cultures relative to that obtained in the absence of the drug. While the number of non-neural cells was markedly depleted in cultures containing ara C, neurite extension was enhanced both in the controls and in the ganglia maintained in 5 mM TREA.

TABLE 1

Neurite Extension in Chick Embryo Ganglia

| Test Sample | | Fiber Index (Numbers of ganglia used in each experiment are shown in parentheses.) | | |
|---|---|---|---|---|
| | | Mean | S.D. | p value vs. Control* |
| Control | | 1.53 (16) | 0.74 | - |
| NGF | $10^{-8}$ M | 4.42 (19) | 1.07 | 0.001 |
| TREA | 2.5 mM | 3.07 (28) | 1.03 | 0.001 |
| | 5.0 mM | 3.42 (32) | 1.21 | 0.001 |
| HEPES | 2.5 mM | 2.66 (18) | 1.17 | 0.001 |
| | 5.0 mM | 3.18 (29) | 0.89 | 0.001 |
| TRIS | 2.5 mM | 2.55 (17) | 0.86 | 0.001 |
| | 5.0 mM | 2.55 (17) | 0.61 | 0.001 |
| Ara C | 27 M | 3.10 (45) | 0.72 | 0.001 |

* In unsupplemented culture medium, which contained 85% Dulbecco's Modified Eagle's medium, 10% dialyzed fetal bovine serum, 3% glucose, 1% glutamine, and 1% penicillin - streptomycin mix.

-9-

## Example 2: The Effects of TREA and Cytosine Arabinoside On Regeneration After Spinal Cord Injury in the Rat

Much current data leave little question that there is an inherent capacity of the injured mammalian central nervous system to undergo some growth and repair. However, formation of astrocytic and connective tissue scars and progressive necrosis are serious impediments to effective regeneration and reinstitution of function. All past efforts to develop treatments that will stimulate tissue repair and regeneration have been unsuccessful because of failure to correct the complex and incoordinated histopathological response of the spinal cord to injury. We now have adopted a more systematic approach in which a histologically-reproducible model of spinal cord injury is utilized and treatments are employed which bring back into balance the relationships between the nerves and their supporting cells, the glial and ependymal cells, and connective tissue elements.

### Method

The dura was opened and a polyethylene tube was sutured to the vertebral spines and adjacent soft tissues so that the opening in one end lay directly over the injured part of the spinal cord. The tubing was brought through a subcutaneous tunnel so that its other end emerged at the base of the skull. A syringe adapter was attached to the external opening for injecting the drugs. In preliminary experiments it was ascertained that if the dura was opened within two days of a crush injury, edema was still present and resulted in a herniation-like protrusion of the substance of the cord and damage to this fragile tissue. Accordingly, we adopted the procedure of waiting two days after crushing the cord, at which time we reoperated on the animal, opened the dura, implanted the

-10-

tube, and commenced the treatment. The drugs were administered four times a day in volumes of 0.5 ml which was found sufficient to thoroughly flood the site of injury. All experiments were done on a double blind basis and three animals each were respectively treated with 10 millimolar TREA; 6 micromolar cytosine arabinoside; 10 millimolar TREA containing 6 micromolar cytosine arabinoside; and the buffered saline vehicle. Treatment of every animal was continued for 14 days, after which the animals were killed and histological sections prepared. The histological preparations were independently evaluated by three scientists experienced in this field.

Results

The results of the study were remarkably consistent. In every case the drug treated animals showed remarkably greater invasion of the lesion by nerve fibers than did the vehicle treated control animals. In fact, there was no obvious difference between the saline-treated control specimens and the untreated animals. In the drug-treated animals, the nerve fibers grew into the lesion site in such profusion that they were no longer oriented longitudinally, but grew rather haphazardly in all directions. Fibers were frequently undulating and varicose and were often arranged in small bundles containing 3-6 axons. The axons were very fine in caliber, most of them being 1-4 microns in diameter. Since they were so close to the resolution of the light microscope, we suspect that considerably greater invasion would be seen by electron microscopy. When the slides were coded and randomized, there was no difficulty in distinguishing between the specimens from the drug-treated and the vehicle-treated animals.

The most prolific nerve growth occurred in the animals treated with cytosine arabinoside and TREA. The animals

-11-

treated with either TREA or cytosine arabinoside also exhibited extensive nerve growth in comparison to the control.

Examples 3-8 detail the preparation of pharmaceutical preparations for use in the present invention.

## Example 3:

A composition is prepared as follows:

100 ml sterile isotonic saline solution

8.5 micromolar cytosine arabinoside

Nerve regeneration is promoted by thoroughly bathing the injury site with the foregoing composition.

## Example 4:

A composition is prepared having the following ingredients:

100 ml sterile isotonic saline solution

0.1 mg hydroxyurea

The foregoing composition promotes regeneration of damaged nerve tissue when administered directly to the site of the injury.

## Example 5:

A pharmaceutical composition is prepared having the following ingredients:

100 ml sterile isotonic saline solution

0.2 mg 5-fluorouracil

Nerve regeneration is facilitated when this composition is administered to damaged nerve tissue in quantities sufficient to bathe the injury site.

-12-

## Example 6:

Pharmaceutical compositions are prepared by adding to each of the compositions of Examples 3-5:

0.3 millimoles HEPES

The nerve regeneration activity of each composition is superior to the activity of each composition without the addition of HEPES.

## Example 7:

Pharmaceutical compositions are prepared by adding to each of the compositions of Examples 3-5 the following:

0.3 millimoles TREA

The ability of each of these compositions to facilitate nerve tissue regeneration is superior to the activity of each composition without TREA.

## Example 8:

A pharmaceutical composition is prepared as follows:

100 ml sterile isotonic saline solution

0.3 millimoles of TREA

This composition, when administered to damaged nerve tissue in a living mammal, promotes nerve tissue regeneration.

## Example 9:

Pharmaceutical compositions are prepared by adding to each of these compositions of Examples 3-7 the following:

0.1 ml 3% hydrogen peroxide solution.

Each of the compositions, when administered to damaged nerve tissue in a living mammal, promotes nerve regeneration.

-13-

Although the foregoing invention has been illustrated by specific embodiments, various modifications and additions are encompassed by the present invention. Accordingly, the scope of this invention is intended to be measured only by the claims which follow and reasonable equivalents thereof.

-14-

1. A pharmaceutical composition for use in a method of promoting nerve regeneration comprising: a pharmacologically- accepted carrier; and an effective amount of an antimitotic agent.

2. The composition of Claim 1, further comprising a buffer providing said composition with a pH of between 7 and 8.

3. The composition of Claim 2, wherein the concentration of said buffer is between about 0.5 and about 20 millimolar.

4. The composition of Claim 2 or 3, wherein the concentration of the unprotonated species of said buffer is between about $1 \times 10^{-5}$M and $1 \times 10^{-3}$M.

5. The composition of Claim 2 to 3, wherein the concentration of the unprotonated species of said buffer is about $2-8 \times 10^{-4}$M.

6. The composition of any one of Claims 2 to 5, wherein said buffer is ACES, ADA, AEPD, AMP, AMPD, BES, BICINE, BIS-TRIS, BIS-TRIS PROPANE, DEA, EPPS, HEPES, MEA, MES, MOPS, PIPES, TAPS, TES, TREA, TRICINE, or TRIS.

7. The composition of any one of Claims 1 to 6, further comprising an oxygen-supplying compound.

8. The composition of Claim 7 wherein the oxygen-supplying compound comprises between about 0.002% and about 0.005% $H_2O_2$ by volume.

9. The composition of any one of Claims 1 to 8 wherein said antimitotic agent is cytosine arabinoside, 5-fluorouracil, or hydroxyurea.

10. The composition of any one of the preceding claims wherein the amount of antimitotic agent is sufficient to reduce the rate of growth of glial cells to the extent that glial cell growth does not prevent nerve tissue growth.

11. The composition of any one of the preceding claims wherein the concentration of said antimitotic agent is between about 3 and about 20 micromolar antimitotic agent.

12. Use of an antimitotic agent for preparation of a composition for promoting nerve regeneration, comprising combining said antimitotic agent with a pharmacologically-accepted carrier.

13. The use of an antimitotic agent according to Claim 12, further including combining said antimitotic agent with a buffer providing said composition with a pH of between 7 and 8.

14. The use of an antimitotic agent according to Claim 13 as applied to the preparation of a composition according to any of Claims 3 to 6.

15. The use of an antimitotic agent according to any one of Claims 12 to 14, comprising further combining said antimitotic agent with an oxygen-supplying compound.

16. The use of an antimitotic agent according to Claim 15, wherein said oxygen-supplying

-16-

compound comprises between about 0.002% and about 0.005% $H_2O_2$.

17. The use of an antimitotic agent according to any one of Claims 12 to 16, wherein said antimitotic agent is cytosine arabinoside, 5-fluorouracil, or hydroxyurea.

18. The use of an antimitotic agent according to any one of Claims 12 to 17, wherein the amount of antimitotic agent is sufficient to reduce the rate of growth of glial cells to the extent that glial cell growth does not prevent nerve tissue growth.

19. The use of an antimitotic agent according to any one of claims 12 to 18, wherein the concentration of said antimitotic agent is between about 3 and about 20 micormolar antimitotic agent.

20. Use of a composition according to any one of Claims 1 to 11 for promoting regeneration of nerve tissue.

21. A pharmaceutical composition for use in a method of promoting nerve regeneration comprising: a pharmacologically-accepted carrier, and a buffer providing said composition with a pH of between 7 and 8.

22. The composition of Claim 21, wherein the concentration of said buffer is between about 0.5 and about 20 millimolar.

23. The compositon of Claim 21 or 22, wherein the concentration of the unprotonated species

-17-

of said buffer is between about $1 \times 10^{-5}$M and $1 \times 10^{-3}$M.

24. The composition of Claim 21 or 22, wherein the concentration of the unprotonated species of said buffer is about $2-8 \times 10^{-4}$M.

25. The composition of any one of Claims 21 to 24, wherein said buffer is ACES, ADA, AEPD, AMP, AMPD, BES, BICINE, BIS-TRIS, BIS-TRIS PROPANE, DEA, EPPS, HEPES, MEA, MES, MOPS, PIPES, TAPS, TES, TREA, TRICINE, or TRIS.

26. The composition of any one of Claims 21 to 25, further comprising an oxygen-supplying compound.

27. The composition of Claim 26, wherein said oxygen-supplying compound comprises hydrogen peroxide.

28. Use of a composition according to any one of Claims 21 to 27 for promoting regeneration of nerve tissue.

29. Use of an antimitotic agent for the manufacture of a medicament for promoting extension of neuritic processes.

30. Use according to Claim 29, wherein said medicament comprises a composition according to any of Claims 1 to 11.

31. Use of a buffer for the manufacture of a medicament for promoting extension of neuritic processes.

32. Use according to Claim 31, wherein said medicament comprises a composition according to any of Claims 21 to 27.

33. A method of promoting the growth of nerve tissue in vitro which comprises treating said tissue with a composition according to any of Claims 21 to 27.